Europäisches Patentamt

European Patent Office

Office européen des brevets

(19)

(11) Publication number: 0 328 372
A2

# (12) EUROPEAN PATENT APPLICATION

(21) Application number: 89301214.6

(22) Date of filing: 08.02.89

(51) Int. Cl.⁴: G 01 N 33/68
G 01 N 33/543

(30) Priority: 08.02.88 US 153704

(43) Date of publication of application:
16.08.89 Bulletin 89/33

(84) Designated Contracting States: FR GB IT

(71) Applicant: BOSTON BIOMEDICAL RESEARCH
INSTITUTE, INC.
20 Staniford Street
Boston, MA 02114 (US)

(72) Inventor: Codington, John F.
1725 Commonwealth Avenue
West Newton Massachussets 02165 (US)

Deh, Mercy E.
54 Mercer Street
Somerville New Jersey 08876 (US)

Davison, Peter F.
193 Cedar Street
Lexington Massachussetts 02173 (US)

(74) Representative: Deans, Michael John Percy et al
Lloyd Wise, Tregear & CO. Norman House 105-109 Strand
London WC2R OAE (GB)

(54) Method for determining antigen.

(57) To determine an antigen in a fluid sample an antibody to the antigen is first provided. The fluid sample is contacted with the antibody so that the antigen in the sample forms an immuno-complex with the antibody. A lectin is contacted with the antigen so that the lectin binds to the antigen. Lectin-bound immunocomplexed antigen is separated from any of the lectin not bound to the immunocomplexed antigen and from the fluid sample. The lectin-bound immunocomplexed antigen is detected. A method is also described for extracting a glycoprotein from a clinical sample by contacting the sample with perchloric acid, centrifuging to provide a first supernatant, contacting the first supernatant with potassium bicarbonate, and centrifuging to provide a second supernatant containing the glycoprotein.

EP 0 328 372 A2

## Description

## METHOD FOR DETERMINING ANTIGEN

This invention relates to determining an antigen in samples of body fluid.

G.S. David et al., 1983, U.S. Patent No. 4,376,110 discloses a "sandwich" immunometric assay for determining an antigen in a fluid, in which the unlabelled polyclonal antibody bound to the solid support and the antibody used as the labelled antibody are replaced by at least one and usually two or more different monoclonal antibodies.

J.F. Codington et al., 1979, Isolation and Purification of an Epiglycanin-Like Glycoprotein From a New Non-Strain Specific Subline of TA3 Murine Mammary Adenocarcinoma, J.N.C.I., vol. 63, no. 1, pp. 153-161, as one step in their method for isolation of epiglycanin-like glycoprotein from mouse ascitic fluid. added perchloric acid to the ascites fluid for "[p]recipitation of extraneous proteins and glycoproteins". Epiglycanin-like glycopeptide, as used herein, means a mammalian epiglycanin and any glycopeptide or glycoprotein capable of forming an immunocomplex with a monoclonal antibody to mouse epiglycanin.

Cells of allotransplantable ascites sublines of the strain A mouse TA3 mammary carcinoma cell line express large quantities of epiglycanin on their surface. J.F. Codington et al., 1983, Cell Surface Macromolecular and Morphological Changes Related to Allotransplantability in the TA3 Tumor, Biomembranes, vol. 11, pp. 207-258. J.F. Codington et al., 1984, Antibody to Epiglycanin and Radioimmunoassay to Detect Epiglycanin-Related Glycoproteins in Body Fluids of Cancer Patients, J.N.C.I., vol. 73. no. 5, pp. 1029-1038, induced anti-epiglycanin polyclonal antibodies by injecting ascites cells of these sublines into rabbit and mouse, and reported the presence in body fluid from human patients, particularly those having metastatic cancer, of glycoprotein that binds to anti-epiglycanin polyclonal antibody.

In general, in one aspect, the invention features a method for determining an antigen in a fluid sample, including providing an antibody to the antigen, contacting the fluid sample with the antibody, so that the antigen in the sample forms an immunocomplex with the antibody, contacting a lectin with the antigen, so that the lectin binds to the antigen, separating the lectin-bound immunocomplexed antigen from any of the lectin not bound to the immunocomplexed antigen and from the fluid sample, and detecting the lectin-bound immunocomplexed antigen.

The method of the invention provides a highly selective and sensitive quantitative lectin immunoassay for an antigen in a clinical sample.

The term lectin, as used herein, means any protein or glycoprotein of non-immune origin (e.g., not an antibody) capable of binding specifically to carbohydrate structures present in proteins.

In preferred embodiments, the antibody is bound to a support and the lectin is labelled; or the lectin is bound to a support and the antibody is labelled; the fluid sample is contacted with the antibody and the lectin concurrently; the lectin is peanut lectin; the labelled lectin is a lectin covalently bonded to a detectable marker, such as an enzyme (for example, horseradish preoxidase or alkaline phosphatase), directly or via a linkage complex including -biotin-avidin-biotin-.

The term "avidin", as used herein, means avidin and avidin derivatives and other avidin-like substances, such as streptavidin, that strongly bind biotin; and "biotin" means biotin and biotin derivatives.

In other preferred embodiments, the antigen is an epiglycanin-like glycoprotein; the antibody binds with epiglycanin; the antigenic site at which the antibody binds with the epiglycanin includes a glycopeptide moeity containing a Galβ1-3GalNAc chain, bonded to a Threonine or a Serine residue on the glycopeptide moeity.

In another aspect the invention features a method for extracting a glycoprotein from a clinical sample, including contacting the sample with a mixture of ammonium acetate and a nonionic detergent, contacting the sample with perchloric acid, centrifuging to provide a first supernatant, contacting the first supernatant with potassium bicarbonate, and centrifuging to provide a second supernatant containing the glycoprotein.

Typically, antigen in a clinical sample such as a serum sample is present both as "free" antigen (antigen not immunocomplexed with antibody) and as "complexed" antigen (immunocomplexed with antibody). We have discovered that, where the antigen of interest is a mucin-type glycoprotein having a large proportion of carbohydrate, extracting the antigen from the sample using perchloric acid ("$HClO_4$") yields an increased quantity of antigen in the supernatant. Extraction by the above method results in dissociation of complexed antigen in the sample from the antibody and precipitation of the antibody, leaving both free and dissociated antigen in the supernatant. Because $HClO_4$ forms a highly insoluble precipitate when mixed with potassium bicarbonate, $HClO_4$ can be readily removed from the supernatant. The supernatant can then be further analysed for determination of total (= free + dissociated) antigen.

In another aspect, the invention features a method for determining the concentration of a glycoprotein antigen in a sample, including an perchloric acid extraction followed by an enzyme-linked lectin immunoassay or a biotin-avidin-biotin-enzyme-linked lectin immunoassay.

In another aspect the invention features a method for detecting an epiglycanin-like glycoprotein in a sample of a body fluid, including contacting the sample with a monoclonal antibody that cross-reacts with a glycopeptide moeity that includes a Galβ1-3GalNAc chain bonded to the glycopeptide moeity, and detecting immunocomplexes of the monoclonal antibody with the sample; and a kit for carrying out the method.

The monoclonal antibodies to epiglycanin can be produced by hybridomas made by immunizing an animal by injection with asialoepiglycanin, and fusing spleen cells from the animal with myeloma cells, as described in European Patent Application No. 88306097.2 (EP-A-0297930) The General Hospital Corporation, the disclosure of which is to be regarded as herein incorporated reference. Examples of such hybridomas include cell lines deposited in the ATCC and having accession numbers ATCC HB9466, HB9467, and HB9468. The epitope for immunocomplexes formed between such monoclonal antibodies and epiglycanin includes a glycopeptide moiety containing a Galβ1-3GalNAc chain; the chain is apparently linked to either a serine or a threonine residue on the glycopeptide.

Other advantages and features of the invention will become apparent from the description of the preferred embodiments.

Two embodiments are described, a biotin-avidin-biotin-alkaline phosphatase-linked lectin immunoassay, and an enzyme-linked lectin immunoassay. The procedures are generally alike up to and including a sample-incubation step; thereafter they diverge as indicated in the descriptions.

Generally, the assay for an antigen of interest is carried out as follows. The surface of a support such as a well plate is coated with an antibody specific for the antigen of interest by allowing a solution containing the antibody to stand for a time on the surface, and then unattached antibody is washed away. Preferably a monoclonal antibody specific for a particular epitope on the antigen is used.

Then non-specific sites on the support surface are blocked by allowing a solution containing a blocking protein such as bovine serum albumin to stand for a time on the coated surface, and unattached blocking protein is washed away.

Then the sample suspected to contain the antigen of interest is diluted with a BSA solution and the diluted sample is allowed to incubate on the coated surface for a time to allow any of the antigen to form immunocomplexes with the antibody on the surface of the support at the specific epitope, and then the sample is washed away, leaving any immunocomplexed antigen on the surface.

## General Description

### Biotin-avidin lectin immunoassay

This embodiment may be understood as an enzyme-linked lectin immunoassay in which the enzyme (alkaline phosphatase) is linked to the lectin via a linkage comprising -biotin-avidin-biotin-.

After the sample has been washed away, leaving any bound antigen on the surface, a solution containing a biotin-conjugated lectin capable of binding with the antigen at a site not masked by or occupied by the antibody is allowed to stand on the coated surface to permit binding of the lectin, and hence of the biotin, to any antigen that may be bound to the antibody on the surface. Then unbound lectin-biotin conjugate is washed away.

Then an avidin-biotin-alkaline phosphatase ("ABP") conjugate is allowed to stand on the coated surface to permit the avidin in the ABP conjugate to bind to the lectin-conjugated biotin bound to the antigen on the surface, and unbound ABP conjugate is washed away.

Then a substrate for alkaline phosphatase is allowed to stand on the coated surface for a time sufficient to permit the substrate to react with the enzyme. Finally, the quantity of the colored reaction product is determined by measuring optical density, as a measure of the quantity of antigen present in the sample.

### Enzyme-linked lectin immunoassay

After the sample has been washed away, leaving any bound antigen on the surface, a solution containing a labelled lectin capable of binding with the antigen at a site not masked by or occupied by the antibody is allowed to stand on the coated surface to permit binding of the lectin, and hence of the label, to any antigen that may be bound to the antibody on the surface. Preferably the label is an enzyme such as horseradish peroxidase, conjugated to the lectin. Then unbound labelled lectin is washed away.

Finally, the quantity of label bound to the plate is detected as a measure of the quantity of lectin, and hence of antigen, complexed with antibody attached to the plate, providing a quantitative measure of the concentration of the antigen in the sample. Where the label is an enzyme, the label may be detected by contacting the labelled-lectin bound surface with a specific substrate for the enzyme, and then measuring a reaction product of the enzyme, as is well-known in the art. Suitable substrates for horseradish peroxidase include 2,2'-azino-bis(3-ethylbenzthiazolinesulfonic acid) ("ABTS") or o-phenylenediamine, each of which yields a colored reaction product which may be quantitatively determined by measuring optical density.

Our methods are described in more detail in the following examples, illustrating use of the methods, employing anti-epiglycanin monoclonal antibodies, for quantitative determination of the glycoprotein antigen epiglycanin.

The monoclonal antibodies to epiglycanin can be prepared by procedures based on the technique developed by Kohler and Milstein, 256 Nature 495 (1975). Generally, somatic cell hybrids or hybridomas are formed in a selective medium by fusing cells from a culture of continuously dividing ("immortal") tumor cells with spleen cells or lymph cells harvested from an animal immunized to the chosen epitope; myeloma cells are killed by the selective medium, and splenocytes or lymphocytes eventually die at the end of their natural life span, leaving only the hybridomas. Each hybridoma is an immortal cell line capable of continuously secreting an antibody specific to a single epitope, by maintenance of the cell line in tissue culture or by injection and ascites formation in vivo.

For production of monoclonal antibodies to epiglycanin, the chosen epitope is on the epiglycanin molecule. Mice are immunized by repeated subcutaneous ("sc") or intraperitoneal ("ip") injection of asialoepiglycanin, and spleen cells from each mouse are fused with mouse myeloma cells to form hybridomas. The hybridomas are then cloned and grown in ascites form in mice for monoclonal antibody production. The monoclonal antibodies are then purified by methods known in the art, as reviewed in Goding, Monoclonal Antibodies: Principles and Practice, Academic Press, New York (2nd Edition, 1986).

A more detailed outline of a protocol for obtaining monoclonal antibodies to epiglycanin is set out below.

Production and purification of asialoepiglycanin

Mouse tumor cells expressing epiglycanin at their cell surfaces are capable of progressive growth in other mice of the same species (allogeneic hosts) or in animals of other species (xenogeneic hosts), and after a period of such growth, epiglycanin appears in the host's body fluids. Ascites cells of the TA3-Ha subline of the strain A mouse TA3 mammary carcinoma, described in Cooper et al., 63 J. Natl. Cancer Inst. 163 (1979), maintained by serial passage in commercially available strain A/WySn mice, are injected (ip) into A/WySn mice, and epiglycanin is isolated from the host ascites fluid 7 days later and purified by column chromatography by techniques described in Cooper et al., id. Sialic acid is removed from the purified epiglycanin by heating a solution of the epiglycanin in 0.05 $\underline{M}$ sulfuric acid at 80° for 60 minutes, and the mixture is dialyzed against hemagglutination buffer, pH 7.0.

Preparation and purification of monoclonal antibodies to epiglycanin

Myeloma and spleen cell fusions giving a good yield of anti-epiglycanin antibody producing clones are made by multiply injecting a commercially available strain C57BL mouse with asialoepiglycanin (prepared as described above) and fusing spleen cells from this mouse with SP2/0-Ag14 ("SP2"; available from the ATCC) mouse myeloma cells. Immunization with asialoepiglycanin gives an enhanced immune response as compared with immunization with epiglycanin.

The monoclonal antibodies are purified by methods known in the art, including generally the following steps (1) precipitation in 50 percent saturated ammonium sulfate; (2) washing of the pellet with 50 percent saturated ammonium sulfate; and (3) fractionation of the dissolved and dialyzed solution on DEAE cellulose (Sigma), or fractionation of the non-dialyzed solution on a column of Sephadex G-200 (Pharmacia) with 0.20 $\underline{M}$ NaCl, pH 7.5, as eluent.

Antigenic determinants on the monoclonal antibodies

Treatment with periodate or with endo-α-N-acetyl-D-galactosaminidase (prepared from $\underline{Diplococcus}$ $\underline{pneumoniae}$) removes the capacity of the antibodies to bind to epiglycanin, suggesting that the epitope is a glycopeptide moeity containing a Galβ1-3GalNAc chain. Endo-α-N-acetyl-D-galactosaminidase is capable of cleaving only the disaccharide 2-acetamido-2-deoxy-3-O-β-$\underline{D}$-galactopyranosyl-α-$\underline{D}$-galactopyranose, which is linked to serine or threonine in epiglycanin.

The monoclonal antibodies bind only weakly to antifreeze glycoprotein, which possesses multiple carbohydrate chains of the same structure as the epiglycanin disaccharide, suggesting that the binding sites for these antibodies require peptide linkages in addition to the carbohydrate chain.

Perchloric acid treatment of serum samples

The procedure is described for a serum sample of 100 μl and for tubes that will accommodate such small samples.

In a conical plastic tube (Sarstedt 4.5 ml tube #57.477) 80 μl of 0.10 $\underline{M}$ ammonium acetate, with 0.05% Tween 20 ("AA-$\overline{Tw}$"), is admixed with 100 μl of serum. Then the mixture is cooled in an ice-water bath, and 20 μl of cold 4.5 $\underline{M}$ perchloric acid ("HClO$_4$") is added. This mixture is agitated using a Vortex mixer on five occasions over a 15 minute period at 0°-3°C, and then centrifuged at 5000 $\underline{g}$ for 10 minutes in the cold. The supernatant, 150 μl, is withdrawn and placed in a similar plastic tube in an ice-water bath, and, beginning promptly, 30 μl of cold 2.5 $\underline{M}$ KHCO$_3$ is added sufficiently gradually to prevent excessive foaming. This mixture is agitated using the Vortex mixer as before, then allowed to stand at 0°-3°C for 15 minutes. Then the mixture is centrifuged (to remove the KClO$_4$ precipitate) at 2000 $\underline{g}$ for 10 minutes in the cold, and finally the clear nearly colorless supernatant is withdrawn and placed in a clean tube.

The biotin-avidin lectin immunoassay

Wells of a 96-well plate (U-shaped wells; NUNC 4-49824) are coated by allowing 100 μl of a solution of monoclonal antibody to epiglycanin ("mAb") (5 to 10 μg/ml) in phosphate-buffered saline ("PBS"), pH 8.0, containing 0.5% sucrose to stand in each well for 16-20 hours at 0°-4°C. Preferably, the PBS is made by dissolving the following in 2.5 ml distilled-deionized water:

| | |
|---|---|
| NaCl | 23.0 g |
| Na$_2$HPO$_4$ | 2.4 g |
| KH$_2$PO$_4$ | 0.64 g, |

then checking the pH and adjusting to pH 8.0 using H$_3$PO$_4$ or NaOH as required, and then bringing the solution to a volume of 3.0 liters by addition of distilled-deionized water. The PBS (as well as other mixtures made with it) should be kept at 4°C, and if it is to be stored more than a few days, it should first be autoclaved. Preferably the PBS-sucrose is made by dissolving 500 mg sucrose in 100 ml PBS, made as described above. The mAb solution is then withdrawn and the wells are washed 5 times with PBS. Nonspecific binding sites on the wells are blocked by allowing 200 μl of a 2% solution of bovine serum albumin ("BSA") in water to stand in each well at 37°C for 45 minutes with shaking. Preferably the 2% BSA blocking solution is made by dissolving 1.0 g crystalline BSA in 50 ml distilled-deionized water. The BSA blocking solution should be kept at 4°C. Then the BSA solution is removed and the wells are washed 5 times with PBS.

Epiglycanin standard solutions (100 μl) at known concentrations, or samples of serum suspected to contain epiglycanin-like material, treated with perchloric acid as described above and diluted with 0.3% BSA in PBS, are added to each well and allowed to incubate for 2 hours at 37°C with shaking. Preferably the BSA-PBS is made by dissolving 300 mg crystalline BSA 100 ml PBS, made as described above.

Preferably the standard solutions of epiglycanin are made from a stock solution prepared as follows. A precisely-measured weight of epiglycanin is dissolved in 10 ml of normal human serum in a 50 ml conical centrifuge tube, and 8.0 ml AA-Tw is admixed. Antigen is then extracted by treating the mixture with $HClO_4$ as described above for perchloric acid treatment of serum samples, with the following adjustments: 2.0 ml of 4.5 $\underline{M}$ $HClO_4$ are used; 2.6 ml of 2.5 $\underline{M}$ $KHCO_3$; centrifugation is in a Sorvall plastic 50 ml tube at 2,000 $\underline{g}$ in a Sorvall RC centrifuge. Using an initial concentration fo 2.4 μg of epiglycanin per ml of serum, this extraction yields an epiglycanin concentration of approximately 1.0 μg/ml. The epiglycanin standard stock solution can be stored in aliquots at 4°C or frozen at -20°C, and diluted with 0.3% BSA-PBS.

After incubation with sample or with epiglycanin standard, the wells are washed 5 times with PBS. To each well is next added 100 μl of solution of peanut lectin conjugated to biotin ("PNA-Biotin", 10 μg/ml) in BSA-PBS. Preferably a PNA-biotin stock solution is prepared by dissolving 1.0 mg of PNA-biotin (E.Y. Labs, # BA 2301) in 10 ml of BSA-PBS, prepared as described above. This PNA-biotin stock solution can be stored in 0.5 ml aliquots in vials at -20°C. The PNA-biotin solution for use is made by mixing one part of the stock solution with ten parts of BSA-PBS.

Incubation with PNA-biotin is allowed to proceed at 37°C for 2 hours with shaking, and then the wells are washed 5 times with PBS. During this incubation an avidin-biotin alkaline phosphatase conjugate ("ABC complex") is prepared by adding 40 μl of avidin solution (approximately 5 μg/ml) to 5.0 ml of PBS and thoroughly mixing. To this thoroughly-mixed solution 50 μl of alkaline phosphatase-conjugated biotin is added with mixing. More preferably the ABC complex is a commercially available preparation (e.g., the Vectastain kit, from Vector Laboratories, Burlingame, CA, and is prepared shortly before use. After 30 minutes 100 μl of this solution is added to each well and allowed to incubate at 37°C for 30 minutes with shaking. The wells are washed 5 times with PBS, and then a substrate is added and allowed to incubate for about 30 minutes with shaking before the reaction is terminated. Any of several suitable substrates can be used, including: phenolophthalein monophosphate (available as a ready-to-use solution from Whittaker Bioproducts, Inc.), and 15 m$\underline{M}$ p-nitrophenyl phosphate in 1.0 m$\underline{M}$ $MgCl_2$ at p$\overline{H}$ 10.3. More preferably the substrate is prepared, shortly before it is to be used, by dissolving 1 tablet of disodium p-nitrophenylphosphate (5 mg enzyme per tablet, Sigma 104) in 6.0 ml substrate buffer. Preferably the substrate buffer is made by dissolving the following in 200 ml distilled-deionized water:

| | |
|---|---|
| $Na_2CO_3$ | 895 mg |
| $NaHCO_3$ | 244 mg, |

then checking the pH and adjusting to pH 10.2-10.3 if necessary. The reaction is stopped by the addition of 100 μl of 0.4 $\underline{M}$ NaOH.

The enzyme-linked lectin immunoassay

Wells of a 96-well flexible plastic plate (U-shaped wells; NUNC 4-49824) are coated with a monoclonal antibody to epiglycanin ("mAb"), by allowing 100 μl of a solution of the mAb (5 to 10 μg/ml) in PBS, pH 7.5, containing 0.5% sucrose, to stand in the wells at 0°-3°C for 16-20 hours. The mAb is then withdrawn, and each well is washed 5 times with PBS.

Non-specific sites in the wells are then blocked by allowing 100 μl of a solution of BSA in water (20 mg per ml of water) to stand in the wells at 37°C for 45 minutes. Then the blocking solution is withdrawn, and the wells are washed 5 times with PBS.

To each well is then added 100 μl of sample diluted in 0.3% BSA; aliquots of serum suspected to contain epiglycanin are added to some wells at dilutions of 1:4, 1:8, and 1:16, while aliquots of an epiglycanin standard at concentrations in a known range, prepared as described above, are added to other wells. Incubation is allowed to proceed at 37°C. for 2 hours. Then the wells are washed 5 times with PBS at pH 7.5.

To each well is then added 100 μl of a solution of Peanut lectin conjugated with horseradish peroxidase in PBS containing 0.3% BSA (2 μg/ml). The enzyme-conjugated peanut lectin is allowed to stand in the plate at 37°C for 2 hours, after which the solution is withdrawn, leaving associated with the support any bound enzyme-conjugated lectin, and each well is washed 5 times with PBS. Finally, a solution of a suitable substrate for the enzyme in a suitable buffer at an appropriate pH with 0.003% hydrogen peroxide (100 μl) is added to each well. Two satisfactory substrates are ABTS (2,2'-azino-bis(3-ethylbenzthiazolinesulfonic acid), 0.1% in citrate-phosphate buffer at pH 6.0; and o-phenylenediamine, 0.1%, in citrate-phosphate buffer at pH 5.0. After 30 minutes the enzyme reactions can be stopped by the use of, respectively, 50 μl of 2.5 $\underline{M}$ hydrofluoric acid or 0.33 $\underline{M}$ citric acid. Optical densities are read in a plate reader at 415 and 493 nm, respectively.

As will readily be recognised by persons skilled in the art, the protocols described above can be varied in their particulars.

For example, other varieties of well plates can be used. Moreover, other forms of support can be used, such as plastic beads.

The sucrose can be omitted from the support-coating step of the immunoassays, the mAb being dissolved in PBS alone. PBS-sucrose is preferred, however, as addition of sucrose aids in reducing background activity while keeping the mAb specificity high.

Other kinds of blocking proteins, well-known in the art, can be used, according to the practice of the particular user.

Any lectin capable of binding the antigen of interest can be used, provided that it binds to a site or sites on the antigen not masked by nor bound by nor complexed with the antibody. The choice of lectin can be made according to the chemistry of the particular antigen of interest.

Other substrates for the horseradish peroxidase or for the alkaline phosphatase can be used, as are well-known. Moreover, other enzymes can be used, such as, for example, β-galactosidase or glucose oxidase. Other means of detecting the immunocomplexed antigen can be used, uncluding other labels, such as, for example, radiolabels or fluorescent labels, although detection by measuring a colored reaction product of an enzyme-substrate reaction is preferred because of its simplicity in use.

The method can be used for determination of any antigen of interest, by substitution of an appropriate antibody to the antigen.

## Claims

1. A method for determining an antigen in a fluid sample, comprising
providing an antibody to the antigen;
contacting said fluid sample with said antibody, so that said antigen in said sample forms an immunocomplex with said antibody,
contacting a lectin with said antigen, so that said lectin binds to said antigen,
separating lectin-bound immunocomplexed antigen from any of said lectin not bound to said immunocomplexed antigen and from said fluid sample; and
detecting said lectin-bound immunocomplexed antigen.

2. The method of claim 1 wherein said antibody is bound to a support.

3. The method of claim 1 wherein said lectin is bound to a support.

4. The method of claim 1 wherein said lectin is labelled.

5. The method of claim 1 wherein said antibody is labelled.

6. The method of claim 1 wherein said fluid sample is contacted with said antibody and said lectin concurrently.

7. The method of claim 1 wherein said antibody is a monoclonal antibody.

8. The method of claim 1 wherein said lectin comprises peanut lectin.

9. The method of claim 4 wherein said labelled lectin comprises a lectin covalently bonded to a detectable marker.

10. The method of claim 9 wherein said detectable marker comprises an enzyme.

11. The method of claim 4 wherein said labelled lectin comprises a lectin covalently bonded to an enzyme via a linkage complex comprising -biotin-avidin-biotin-.

12. The method of claim 10 wherein said enzyme comprises horseradish peroxidase.

13. The method of claim 11 wherein said enzyme comprises alkaline phosphatase.

14. The method of claim 1, wherein said antigen comprises an epiglycanin-like glycoprotein.

15. The method of claim 1 wherein said antibody binds with epiglycanin.

16. The method of claim 15 wherein the antigenic site at which said antibody binds with said epiglycanin comprises a glycopeptide moiety containing a Galβ1-3GalNAc chain.

17. The method of claim 16, wherein said Galβ1-3GalNAc chain is bonded to a Threonine residue on said glycopeptide moiety.

18. The method of claim 16, wherein said Galβ1-3GalNAc chain is bonded to a Serine residue on said glycopeptide moeity.

19. A method for extracting a glycoprotein from a clinical sample, comprising
contacting said sample with perchloric acid,
centrifuging to provide a first supernatant,
contacting said first supernatant with potassium bicarbonate, and
centrifuging to provide a second supernatant containing said glycoprotein.

20. A method for determining a glycoprotein antigen in a clinical sample, comprising
the steps of claim 19, and
the steps of claim 1.

21. A method for determining an epiglycanin-like glycoprotein in a sample of a body fluid, comprising
contacting said sample with a monoclonal antibody that cross-reacts with a glycopeptide moeity,
said moeity comprising a Galβ1-3GalNAc chain bonded to said glycopeptide moeity, and
detecting immunocomplexes of said monoclonal antibody with said sample.

22. A kit for determining an epiglycanin-like glycoprotein in a sample of a body fluid by contacting said sample with a monoclonal antibody that cross-reacts with a glycopeptide moeity comprising a Galβ1-3GalNAc chain bonded to said glycopeptide moeity, and detecting immunocomplexes of said monoclonal antibody with said sample, said kit comprising said monoclonal antibody, and instructions for use.